# EUROPEAN PATENT APPLICATION

(11) **EP 4 183 247 A1**
(43) Date of publication of application: **24.05.2023**
(21) Application number: 21208719.1
(22) Date of filing: 17.11.2021
(51) Int. Cl.: A01H 1/04, G01J 3/28

(54) **METHOD AND APPARATUS FOR SORTING SEEDS**

(71) Applicant: KWS SAAT SE & Co. KGaA, 37574 Einbeck (DE)
(72) Inventor: LAGE, Jacob, Thriplow SG8 7RE (GB); UTSCHIG, Christian, 37574 Einbeck (DE); HIRSCHMANN, Christian Bernd, 37574 Einbeck (DE)
(74) Representative: Gulde & Partner

(57) **Abstract**

The present invention relates to a method for categorizing/sorting seeds, the method comprising the steps of: providing (S11) a sample including at least one seed; obtaining (S12) a near infrared, NIR, spectrum of at least a subset of the sample; determining (S14) presence of an organic colorant in at least the subset of the sample based on the obtained NIR spectrum; and categorizing/sorting (S19) at least the subset of the sample based on the determination. Based on this, mis-colored white seed and blue seed with a fading blue color can be properly categorized. Further, it is also possible to distinguish between single blue and double blue seed.

## Description

### Field of the Invention

The present invention relates to an improved method for categorizing/sorting seeds, particularly blue and white seeds in the basic seed production step of a BLue Aleurone (BLA) hybrid cereal system. The present invention is also directed to an apparatus for categorizing/sorting seeds and a corresponding mobile device comprising said apparatus.

### Technological Background

Essential for any hybrid system is the production of male-sterile female parents. WO 92/01366 A1 discloses a male sterility system which allows the maintenance of male sterility that can be used in the production of hybrid cereal plants, in particular hybrid wheat plants. Male sterility can be achieved by possessing a homozygous deletion on the short arm of chromosome 4B in wheat. The deletion typically used is the 'Probus' deletion (Fossati A, Ingold M. 1970. A male sterile mutant in Triticum aestivum. Wheat Inform Serv 30:8-10).

Recently, the ms1 gene located in the region concerned by the deletion has been identified as the causative gene. If this gene is deleted physically or knocked out/down by a mutation or targeted modification (e.g. WO 2016/048891 A1), then a reliable male sterility can be established. Fertility can then be easily restored when a wheat line carrying homozygously the deletion or the mutation/modification, is crossed with any normal wheat. Resulting progenies or hybrids are fertile as the deletion or the mutation/modification is only heterozygously present. Thus, plants or plant lines comprising the above deletion or mutation/modification are suitable for use in producing hybrid plants.

However, in order to maintain the male-sterile female parent further components are needed. As such, WO 92/01366 A1 teaches the use of a male parent that is isogenic to the female but having an alien addition chromosome bearing a dominant male fertility restorer gene from *Triticum boeticum* on the short arm and the BLue Aleurone (BLA) gene from *Agropyron elongatum* on the long arm, in a cross with the female parent for maintenance of the male sterile female parent, whereby the BLA gene, if expressed, confers a characteristic blue coloration of the progeny seed.

The BLA hybrid cereal system relies on the effect sorting of blue and normal colored seed, wherein the normal colored seed is subsequently referred to as "white" seed but covers the normal spectrum of seed color, produced during the basic seed production stage. Therein, blue seed is planted, and a mix of blue and white seed is harvested. The white seed will grow into male sterile seed and become the female component of hybrid seed production. For maintenance breeding, the blue seed can be replanted for another round of blue-white seed production. The color of the blue seed is caused by a blue color marker, which is co-expressed with a fertility-related gene.

According to the prior art, the blue and white seed can be mechanically separated using commercially available seed sorters equipped with optical cameras that detect the seed color. However, normal environmental conditions can result in the white seed becoming mis-colored, and blue seed can lose some of their distinct blue color. Either of these can result in difficulties when a separation is made based on visual light spectrum.

The solutions of the prior art lead to very poor separation efficiency and several rounds of separation may be required to achieve purely separated seed pools. Seed purity directly affects the quality of seed breeding and subsequent processing products. For example, in the process of seed harvest and storage, the impurities or hybrids may be mixed in the normal seed, which results in the economic losses to agricultural production and processing. Therefore, it is crucial to sort impurities and hybrids to ensure that the seed purity meets the market criteria.

Furthermore, due to genetic defects, unwanted dark blue seed may be generated in some cases, which is subsequently referred to as double blue seed. Since such double blue seed cannot be used in breeding pool development, it is desired to discard the same.

It is therefore an object of the present invention to provide a method that allows to reliably detect a difference in blue seed, comprising single blue seed and double blue seed, and white seed regardless of a cereal type and variety.

### Summary of Invention

According to a first aspect, the invention is directed to a method for categorizing/sorting seeds, the method comprising the steps of: providing a sample including at least one seed (single kernel); obtaining a near infrared (NIR) spectrum of at least a subset of the sample; determining presence of an organic colorant in at least the subset of the sample based on the obtained NIR spectrum; and categorizing/sorting at least the subset of the sample based on the determination.

In contrast to the prior art, which suggests distinguishing between blue seed and white seed using visual light, the present solution uses light in the NIR spectrum to determine whether an organic colorant is present.

NIR spectroscopy is a spectroscopic method that uses the near infrared region of the electromagnetic spectrum (ranging from 780 nm to 2500 nm). Typical applications include medical and physiological diagnostics and research. NIR spectroscopy is based on molecular overtone and combination vibrations. Such transitions are forbidden by the selection rules of quantum mechanics. As a result, the molar absorptivity in the NIR region is typically quite small. One advantage is that NIR can typically penetrate much further into a sample than mid-infrared radiation. NIR spectroscopy is therefore very useful in probing bulk material with little or no sample preparation. Using NIR spectrometry for detecting colorants advantageously allows for identifying a well-defined target marker with quantitative precision in a destruction-free measurement that can be carried out in addition to visual inspection.

Plant pigments are colored substances produced by plants and are important in controlling photosynthesis, growth, and development. (P. Sundhakar et al. Phenotyping Crop Plants for Physiological and Biochemical Traits). In the present specification, the term plant pigment may have the same meaning as organic colorant. The organic colorant may comprise at least one colorant of the group including betalains, carotenoids, anthocyanins, flavonoids, anthraquinone and/or chlorophylls, and others known to the skilled person. For example, the blue seed color may be a result of an anthocyanin in the aleurone layer of a cereal seed, wherein the present invention is not limited thereto. Anthocyanins are, for example, used as natural color pigment for the food industry and are a water-soluble flavonoid compound that is responsible for the appearance of certain colors in nature and may appear - depending on the pH - as red, purple, or blue. However, anthocyanins are susceptible to gradual degradation when exposed to certain food processing methods or even through prolonged/improper storage. Chemical assays exist, which allow to determine a total anthocyanin content but said assays often require destroying the sample. In contrast, spectroscopy-based assays are simple, fast, and non-destructive analytical tools and hence, may be used in determining the total anthocyanin content e.g. in cereal grains. It was surprisingly found that anthocyanin content can be determined with high precision using NIR spectrometry even when gradual degradation impeded visual inspection.

Based on this, the present invention provides an approach for detecting and sorting seed having an elevated level of a certain organic colorant, such as (but not limited to) anthocyanin, which is caused by a blue color marker being co-expressed with a fertility-related gene. Since the presence of an organic colorant - such as anthocyanin - is determined, the present invention is also able to detect when e.g. a blue color fades. This is the case because there is a correlation between NIR spectra and the amount of anthocyanins in seed. Accordingly, by using the NIR spectrum, it is possible to detect a difference in concentration between single blue seed and double blue seed, particularly since the level of anthocyanins in double blue seed is approximately twice as high as in single blue seed.

According to an aspect of the present disclosure, the method may further comprise the step of identifying a signal associated with the organic colorant in the obtained NIR spectrum, wherein the categorizing/sorting of at least the subset of the sample is based on the identified signal. Since the identified signal corresponds to an NIR spectroscopy reflectance measurement, it is possible to determine whether a certain organic colorant is present. Alternatively, an absorption measurement may be likewise conducted to determine whether a certain organic colorant is present. Therein, the identified signal is in the range of 15500 cm⁻¹ to 400 cm⁻¹, including sub-ranges of 15500 cm⁻¹ to 6000 cm⁻¹ and 6000 cm⁻¹ to 400 cm⁻¹. Another aspect of the present disclosure provides that the identified signal is in one of the ranges 15385 cm⁻¹ to 10526 cm⁻¹ (650-950 nm), 8547 cm⁻¹ to 8000 cm⁻¹ (1170-1250 nm), 7519 cm⁻¹ to 7092 cm⁻¹ (1330-1410 nm), 6250 cm⁻¹ to 5917 cm⁻¹ (1600-1690 nm) and 5263 cm⁻¹ to 4167 cm⁻¹ (1900-2400 nm). This is particularly advantageous since wavelengths in these ranges corresponded to the UV/visible absorption bands of organic colorants such as anthocyanin. It has been found that such characteristic NIR signals of organic colorants are reliably detected even when optical inspection is deteriorated. The following table summarized wavelengths, which are applied according to the present invention.

| Wavelength [nm] | Wavenumber [cm-1] |
|---|---|
| 650-950 | 15385-10526 |
| 1170-1250 | 8547-8000 |
| 1330-1410 | 7519-7092 |
| 1600-1690 | 6250-5917 |
| 1900-2400 | 5263-4167 |

According to a further aspect, the method may further comprise the step of: determining an amount of the organic colorant in at least the subset of the sample based on the obtained NIR spectrum, wherein the categorizing/sorting of at least the subset of the sample is based on the determined amount of the organic colorant. Hence, a quantitative determination of colorant is achieved and a distinction between single blue seed and double blue seed, i.e. blue seed with different levels of organic colorants such as anthocyanin, can be made. Further, bulk measurements for quality control can be performed. Therein, low thresholds can be set for the amount of colorant, e.g., for pre-assessing bulk samples for presence of any colorant.

Another aspect of the present disclosure provides that the NIR spectrum is obtained for a bulk sample, wherein the method further comprises the steps of: normalizing the determined amount of the organic colorant with respect to a size of the bulk sample; comparing the normalized amount of organic colorant with a predefined threshold; and categorizing/sorting the bulk sample based on the comparison. In this way, bulk measurements can be carried out for quality control, so that it can be determined that less than a certain percentage or amount of e.g. blue seed, comprising single blue seed and double blue seed, is contained in a bag declared as white seed. The size of the bulk sample is preferably determined as a mass and/or a volume of the bulk sample. The predefined threshold preferably allows for meeting quality standards. It is also possible to measure an overall intensity (i.e. a total amount) of a colorant, for example by determining that ten blue seeds are present in 1000 seeds.

A further aspect of the present disclosure requires the steps of: obtaining a first NIR spectrum of a first subset of the sample and a second NIR spectrum of a second subset of the sample; determining presence of the organic colorant in the first subset by classifying the first NIR spectrum and in the second subset by classifying the second NIR spectrum; categorizing/sorting the first subset and the second subset based on the classification, wherein the classification of the NIR spectrum is performed using at least one of a principal component analysis (PCA), a partial-least-squares-regression (PLS-R) or partial least squares discriminant analysis (PLS-DA) model based on labeled spectra, a trained support vector machine classification (SVM-C) or support vector machine regression (SVM-R) model, a trained artificial neural network (ANN), or a k-nearest neighbors algorithm (k-NN). Using any of these classifications allows to effectively differentiate between samples of a first NIR spectrum and samples of a second NIR spectrum, wherein said spectra pertain to different organic colorants.

According to a preferred aspect, a plurality of NIR spectra is obtained for a plurality of subsets of the sample, wherein presence of the organic colorant is determined for each of the plurality of subsets based on classifying the respective NIR spectrum of the plurality of NIR spectra using at least one of PCA, PLS-R, PLS-DA, SVM-C, SVM-R, ANN, or k-NN, wherein each of the plurality of subsets is categorized based on the respective classification, and wherein each of the plurality of subsets is sorted based on its categorization. Hence, it is possible to distinguish between more than two sample subsets such as single blue seed, double blue seed and white seed. The method of the present disclosure may further comprise the step of: sorting the first subset and the second subset based on the classification, wherein a subset of the sample preferably consists of a single seed.

Another aspect of the present disclosure provides that the method may further comprise the step of: obtaining an image of at least the subset of the sample, wherein the presence of an organic colorant in at least the subset of the sample is determined based on the obtained NIR spectrum and based on the image. By using NIR spectroscopy in combination with visual light, a precision according to which blue and white seed can be detected is further increased. Preferably, the image may be a photometric image. More preferably, the NIR spectroscopy can be used in combination with visible light with more than one camera for sorting the seed. Alternatively, seed will go through more than one cycle of sorting which could be a first cycle with visible light and a second cycle with NIR spectroscopy only. Further, a combination of visible light and NIR spectroscopy may be applied in at least one of the cycles or only NIR spectroscopy may be used in the cycles.

A further aspect of the present invention is to provide a method for precise seed sorting by using NIR in combination with other state of the art sorting methods including visual sorting or imaging sorting (e.g. by combination with at least one camera), x-ray sorting, Raman Spectroscopy and any other suitable spectroscopic methods.

According to a second aspect, the invention is directed to an apparatus for categorizing/sorting seeds, said apparatus comprising: a near infrared (NIR) spectrometer, including a light source configured to emit light with a wavelength in between 650 nm and 2500 nm; a detector configured to detect a NIR spectrum in a range of 15500 cm⁻¹ to 400 cm⁻¹; a sample holder configured to hold a sample relative to the light source and the detector to perform NIR measurements in a transmission and/or reflection mode; and means adapted to execute the steps of the method described above.

According to a further aspect, the apparatus may further comprise an image sensor configured to obtain an image of at least the subset of the sample and means adapted to execute the steps of the above method and/or a feeder system configured to feed at least the subset of the sample to the NIR spectrometer, preferably configured to feed at least the subset of the sample to the NIR spectrometer and the image sensor. However, any suitable sensor may be combined with NIRS to facilitate and improve sorting efficiency as well as for assessing quality parameters with respect to necessary quality check.

According to a preferred aspect, a computer program is provided, said computer program comprising instructions to cause the above apparatus to execute the steps of the above method.

According to a third aspect, the invention is directed to a mobile device comprising the above apparatus.

According to a fourth aspect, the invention is directed to the use of the above method and/or the above apparatus according for categorizing/sorting seeds of a hybrid seed system, the hybrid seed system comprising seeds including an organic colorant.

Another aspect of the present disclosure provides that the hybrid seed system is a hybrid wheat system comprising a monosomic alien addition chromosome carrying a male fertility restorer gene and a color marker gene as a selection marker gene and that the color marker gene is able to confer a characteristic coloration and is co-expressed with a fertility-related gene.

The present invention may be applied during basic seed production as opposed to sorting male seed from hybrid seed, and allows for efficient separation of white and blue seed even when the color difference is not so clear in the visual light spectrum. Further, sorting single blue and double blue seed allows to distinguish the seed based on a concentration of e.g. anthocyanins. By using the NIR spectrum, it is possible to detect a difference between blue seed and white seed regardless of the variety even without using visual light.

Further aspects and preferred embodiments of the invention result from the dependent claims, the drawings and the following description of the drawings. Different disclosed embodiments are advantageously combined with each other if not explicitly stated otherwise.

### Brief Description of the Drawings

The features of the invention become apparent to those skilled in the art by the detailed description of exemplary embodiments with reference to the attached drawings in which:
- Fig. 1: schematically illustrates an embodiment of a method for categorizing/sorting seeds;
- Fig. 2: illustrates results of a principal component analysis (PCA) performed on a sample including a plurality of seeds/kernels;
- Fig. 3: illustrates results of a PCA performed on single seeds/kernels; and
- Fig. 4: schematically illustrates at which stages of a seeding/harvesting cycle the present invention may be applied.

### Detailed Description of the Invention

Reference will now be made in detail to embodiments, examples of which are illustrated in the accompanying drawings. Effects and features of the exemplary embodiments, and implementation methods thereof will be described with reference to the accompanying drawings. In the drawings, like reference numerals denote like elements, and redundant descriptions are omitted. The present invention can be embodied in various different forms, and should not be construed as being limited to only the illustrated embodiments herein. These embodiments are provided as examples so that this disclosure will be complete and will fully convey the aspects and features of the present invention to those skilled in the art.

Accordingly, elements not considered necessary to those having skill in the art for a complete understanding of the features of the present invention may not be described.

As used herein, the term "and/or" includes any and all combinations of one or more of the associated listed items. Further, the use of "may" when describing embodiments of the present invention refers to "one or more embodiments of the present invention." In the following description of embodiments of the present invention, the terms of a singular form may include plural forms unless the context clearly indicates otherwise.

It will be understood that although the terms "first" and "second" are used to describe various elements, these elements should not be limited by these terms. These terms are only used to distinguish one element from another element. For example, a first element may be named a second element and, similarly, a second element may be named a first element, without departing from the scope of the present invention. As used herein, the term "substantially", "about," and similar terms are used as terms of approximation and not as terms of degree, and are intended to account for the inherent deviations in measured or calculated values that would be recognized by those of ordinary skill in the art. Further, if the term "substantially" is used in combination with a feature that could be expressed using a numeric value, the term "substantially" denotes a range of +/- 5% of the value centered on the value.

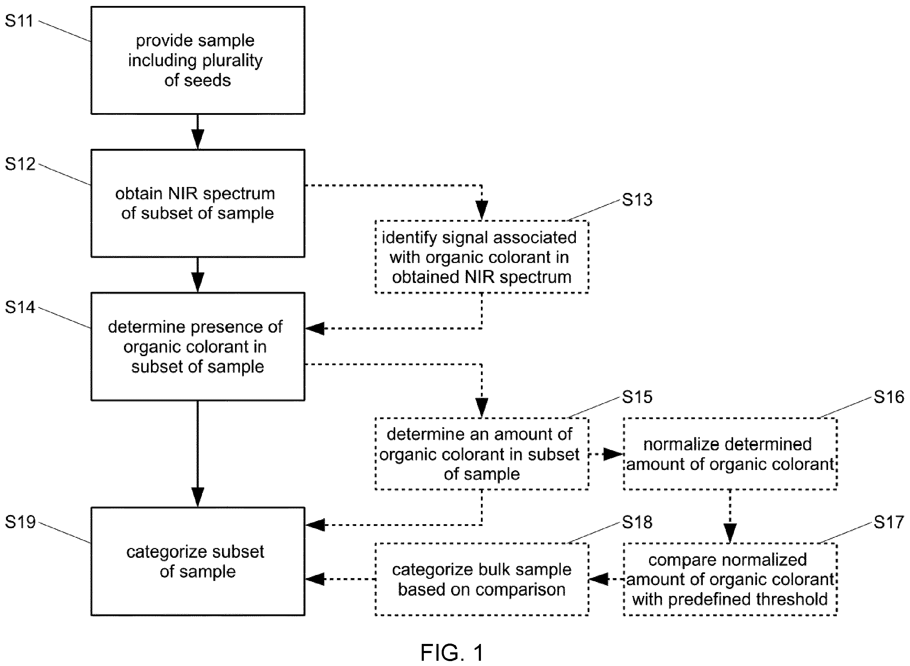

Figure 1 schematically illustrates an embodiment of a method for categorizing/sorting seeds. According to step S11, a sample including a plurality of seeds is provided. Subsequently, according to step S12, a near infrared (NIR) spectrum of at least a subset of the sample is obtained.

Based on the obtained NIR spectrum, presence of an organic colorant is determined in at least the subset of the sample as illustrated in step S14. Finally, according to step S19, the method further comprises categorizing/sorting at least the subset of the sample based on the determination. It has to be noted that steps shown in dashed boxes in figure 1 represent optional steps.

For example, as an optional step S13, it is provided that the method further comprises the step of identifying a signal associated with the organic colorant in the obtained NIR spectrum. By means of this, the categorizing/sorting of at least the subset of the sample is based on the identified signal.

According to a preferred embodiment, the method may further comprise optional step S15 according to which an amount of the organic colorant in at least the subset of the sample is determined based on the obtained NIR spectrum. By means of this, the categorizing/sorting of at least the subset of the sample can be based on the determined amount of the organic colorant.

In case the NIR spectrum is obtained for a bulk sample, the method further comprises normalizing the determined amount of the organic colorant with respect to a size of the bulk sample as illustrated in step S16. Subsequently, according to step S17, the normalized amount of organic colorant is compared with a predefined threshold. After that, a categorization of the bulk sample is performed based on the comparison in step S18.

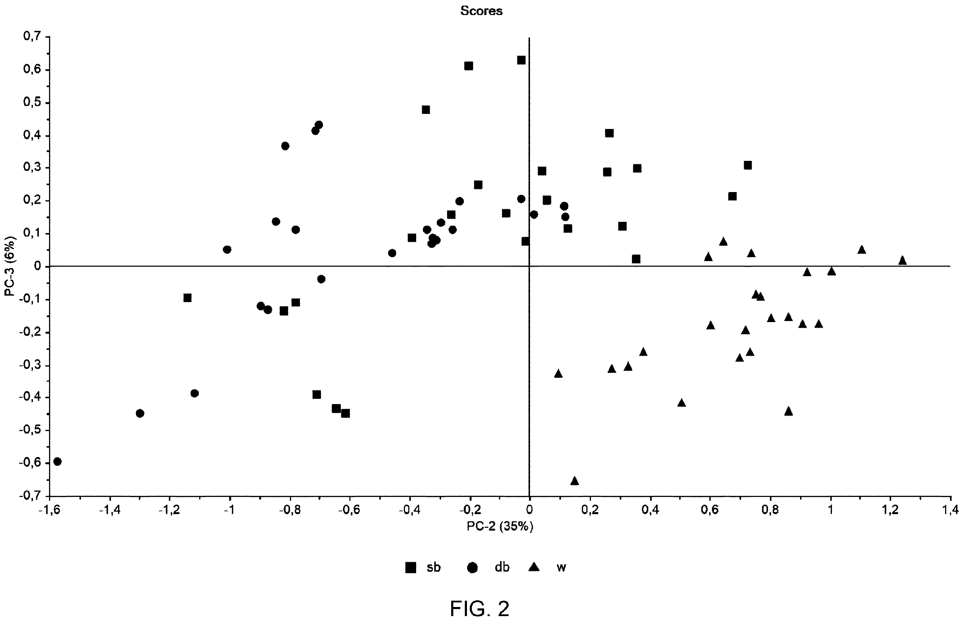

Figure 2 illustrates the results of a principal component analysis (PCA) performed on a sample including a plurality of seeds/kernels. Therein, the scores plot/scores room shows a clear difference between white seed (w), which is represented by triangles, and blue seeds, which is represented by squares (single blue seed; sb) and circles (double blue seed; db).

In general, in order to verify the performance of using NIR spectroscopy to distinguish between different seed, the following set of eight different wheat variety samples was exemplarily chosen, wherein all samples contain white, single blue, and double blue seed.

**Table 1: Sample overview**

| Sample: | Variety: |
|---|---|
| 1 | ClevelandBla1 |
| 2 | GymnastBla3 |
| 3 | KielderBla2 |
| 4 | LiliBla1 |
| 5 | LoftBla1 |
| 6 | SolehioBla1 |
| 7 | SolehioBla4 |
| 8 | TrinityBla1 |

These eight samples were used to develop a method to distinguish, based on NIR reflectance measurements, between white and blue (comprising both, single blue and double blue) seeds on the one hand, and white, single blue and double blue seeds on the other hand.

More specifically, the samples were measured using a Bruker MPA NIRS spectrometer and small beakers comprising 20g to 25g samples, which corresponds to about 500 seeds or kernels. The beakers were placed in a rotating holder of the spectrometer and measured in triplicate each (refilled and remeasured) and thus three spectra were recorded for each sample. The technical specification of the Bruker MPA NIRS spectrometer is given below.

**Table 2: Spectrometer specification**

| Detector type: | Lead sulphide (PbS) |
|---|---|
| Spectral range [nm]: | 800 - 2780 |
| Spectral range [wavenumber]: | 12500 - 3600 |
| Spectral resolution: | 8 cm⁻¹ |
| Number of scans: | 32 |

Furthermore, to minimize scattering and reflection losses, a half sphere was placed on top of the kernels.

As indicated above, all spectra were labelled with a specific code (w, sb, db) according to their color classes. Further, for a regression test, the labels 0, 1 and 2 were provided to the white, single blue, and double blue classes, respectively.

**Table 3: Codes and labels of color classes**

| Sample color: | Labelling approach classification: | Labelling approach regression: |
|---|---|---|
| White | w | 0 |
| Single blue | sb | 1 |
| Double blue | db | 2 |

For the blue and white separation test, the single blue samples as well as the double blue samples were labelled as blue, which leads to the following number of samples for the two different approaches.

**Table 4: Measurement approaches and samples**

| Approach: | Samples white: | Samples blue: | Samples single blue: | Samples double blue: | Samples total: |
|---|---|---|---|---|---|
| White, blue | 8 | 16 | - | - | 24 |
| White, single blue, and double blue | 8 | - | 8 | 8 | 24 |

Referring back to figure 2, the result of the PCA is used to determine the effectiveness/potential of different classification approaches. In particular, a classification of the samples was performed using a partial-least-squares-regression (PLS-R) model and a support vector machine regression (SVM-R) model, wherein no spectra pre-treatment was applied for both approaches.

A PLS-R model requires numbers as input and delivers numbers as predictions. Based on the labels 0 (for white), 1 (for single blue) and 2 (for double blue), predictions were classified using thresholds. For example, a prediction value smaller than 0,5 corresponds to white, a prediction value greater than or equal to 0,5 but smaller than 1,5 corresponds to single blue, and a prediction value greater than or equal to 1,5 corresponds to double blue. Accordingly, the thresholds are chosen in order to decide to which class a predicted spectrum belongs.

As indicated above, the scores plot of figure 2 shows a clear difference between white seeds, which are represented by triangles, and blue seeds, which are represented by squares (single blue seed; sb) and circles (double blue seed; db).

### a) Assemblage of kernels; White and blue; PLS-R

As it is apparent from the below shown table, a blue and white separation test based on the PLS-R, which is a model based on linear regression, shows an accuracy of 100%.

**Table 5: White/blue classification confusion matrix of PLS-R model**

| | | Predicted class | |
|---|---|---|---|
| | | blue | white |
| True class | blue | 48 (100% classified) | |
| | white | | 24 (100% classified) |

The numbers in the confusion matrix show the number of samples classified and misclassified with the respective percentage of classified and misclassified shown in brackets.

According to this example, 48 blue kernels (true class) are properly predicted as blue and 24 white kernels are properly predicted as white.

### b) Assemblage of kernels; White, single blue and double blue; PLS-R

As indicated below, the PLS-R classification model for white, single blue and double blue shows a 100% accuracy in predicting the white samples.

However, the accuracy in predicting single blue and double blue is lower with 91,7% for single blue and 83,3% for double blue. In detail, two single blue kernels were misclassified as double blue and four double blue kernels were misclassified as single blue.

Nevertheless, all white samples were identified as white and all blue samples, including single blue and double blue, were identified as blue samples.

**Table 6: White/single blue/double blue classification confusion matrix of PLS-R model**

| | | Predicted class | | |
|---|---|---|---|---|
| | | single blue | double blue | white |
| True class | single blue | 22 (91,7% classified) | 2 (8,3% misclassified) | |
| | double blue | 4 (16,7% misclassified) | 20 (83,3% classified) | |
| | white | | | 24 (100% classified) |

Within the predicted class for single blue, there is a classification of 84,6% and a misclassification of 15,4% since 22 of the overall 26 samples in this predicted class have been classified correctly.

Further, within the predicted class for double blue, there is a classification of 90,9% and a misclassification of 9,1% since 20 of the overall 22 samples in this predicted class have been classified correctly.

### c) Assemblage of kernels; White and blue; SVM-R

SVM-R uses a support vector which allows a finer classification if there are, for example, non-linearities in the data. The white and blue classification according to the SVM-R model also shows a 100% accuracy.

**Table 7: White/blue classification confusion matrix of SVM-R model**

| | | Predicted class | |
|---|---|---|---|
| | | blue | white |
| True class | blue | 48 (100% classified) | |
| | white | | 24 (100% classified) |

Hence, all blue kernels are properly predicted as blue and all white kernels are properly predicted as white.

### d) Assemblage of kernels; White, single blue and double blue; SVM-R

However, the accuracy in the white, single blue and double blue prediction using SVM-R is not 100% for all classes. Said three-class model shows a 100% accuracy for white and double blue samples and an 83,3% accuracy for single blue samples. This means that 16,7% of the single blue samples were identified as double blue samples.

**Table 8: White/single blue/double blue classification confusion matrix of SVM-R model**

| | | Predicted class | | |
|---|---|---|---|---|
| | | single blue | double blue | white |
| True class | single blue | 20 (83,3% classified) | 4 (16,7% misclassified) | |
| | double blue | | 24 (100% classified) | |
| | white | | | 24 (100% classified) |

Hence, with the PLS model as well as with the SVM-R model, it is clearly possible to distinguish between blue (regardless whether single blue or double blue) and white samples with a 100% accuracy when an analysis is performed on a subset including a plurality of seeds (kernels).

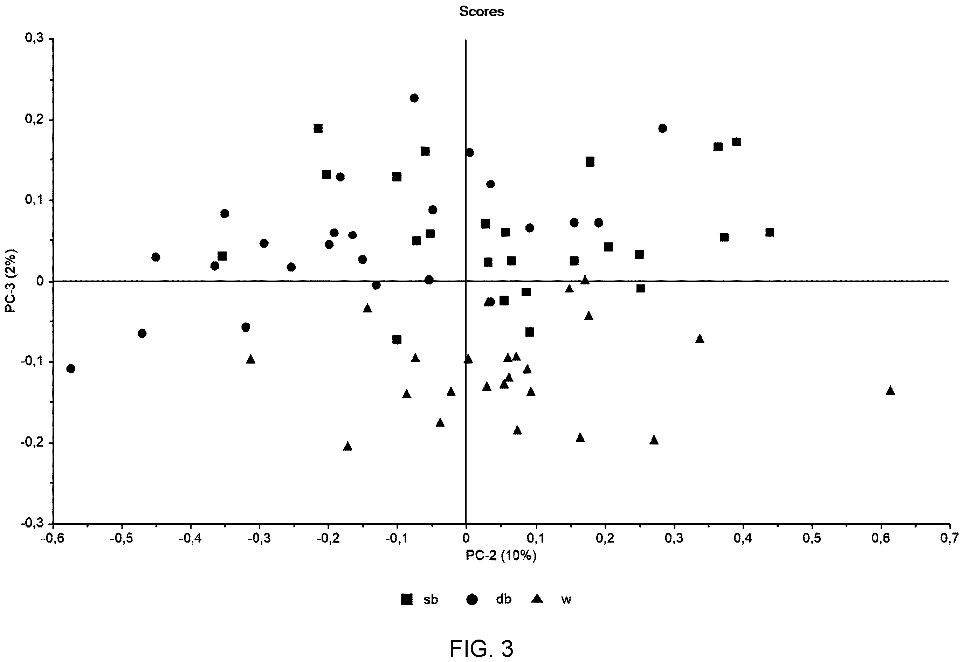

Figure 3 illustrates the results of a PCA performed on single seeds (kernels). Therein, the scores room shows the differences between white and blue kernels (comprising both, single blue and double blue kernels). Since the triangles (corresponding to the white kernels) are located in the lower part of the scores plot, whereas the squares/circles (corresponding to the blue kernels) are located in the upper part of the scores plot, white and blue kernels are visually separated.

However, the separation is not as obvious as in the embodiment of figure 2, which pertains to whole sample measurements, i.e. measurements performed on a subset including a plurality of seeds or kernels. Single blue and double blue kernels are more difficult to separate in comparison to the whole sample measurement of figure 2 although there are differences in the respective spectra.

In order to investigate the performance of the inventive classification based on NIR spectroscopy, both PLS and SVM models were tested.

### e) Single kernel; White and blue; PLS-R

The blue and white classification model based on the PLS-R for single kernels shows the same 100% accuracy as for whole samples, which has been described above in a).

**Table 9: White/blue classification confusion matrix of PLS-R model for single kernel**

| | | Predicted class | |
|---|---|---|---|
| | | blue | white |
| True class | blue | 48 (100% classified) | |
| | white | | 24 (100% classified) |

### f) Single kernel; White, single blue and double blue; PLS-R

However, the PLS-R classification model for white, single blue and double blue shows a lower accuracy when predicting all three classes of kernels.

**Table 10: White/single blue/double blue classification confusion matrix of PLS-R model for single kernel**

| | | Predicted class | | |
|---|---|---|---|---|
| | | single blue | double blue | white |
| True class | single blue | 14 (58,3% classified) | 7 (29,2% misclassified; overall: 41,7%) | 3 (12,5% misclassified; overall: 41,7%) |
| | double blue | 4 (16,7% misclassified) | 20 (83,3% classified) | |
| | white | 4 (16,7% misclassified) | | 20 (83,3% classified) |

For all classes, there are false positive rates ranging from 16,7% (white kernels or double blue kernels being misclassified as single blue kernels) to 41,7% (single blue kernels being misclassified as double blue kernels and white kernels).

Hence, although the white and blue PLS-R classification delivers a 100% accuracy, this model has drawbacks in distinguishing the single kernel samples for the three-class approach.

### g) Single kernel; Single blue and double blue; PLS-R

As it is apparent from the table shown below, a model for the separation of just single blue and double blue kernels does also not deliver better results than the three-class model.

**Table 11: Single blue/double blue classification confusion matrix of PLS-R model for single kernel**

| | | Predicted class | |
|---|---|---|---|
| | | single blue | double blue |
| True class | single blue | 18 (75% classified) | 6 (25% misclassified) |
| | double blue | 5 (20,8% misclassified) | 19 (79,2% classified) |

Said model has accuracies below 80% (i.e. 75% of the single blue kernels being properly classified and 79,2% of the double blue kernels being properly classified) and hence, is slightly inferior to the three-class PLS-R model described above in section f), which has accuracies of 83,3% for both single blue and double blue.

### h) Single kernel; White and blue; SVM-R

The SVM-R model shows a comparable performance in comparison to the PLS-R model described in section e).

In detail, the white and blue classification shows a slightly lower accuracy of 91,7%.

**Table 12: White/blue classification confusion matrix of SVM-R model for single kernel i) Single kernel; White, single blue and double blue; SVM-R**

| | | Predicted class | |
|---|---|---|---|
| | | blue | white |
| True class | blue | 47 (95,8% classified) | 1 (4,2% misclassified) |
| | white | 1 (8,3% misclassified) | 23 (91,7% classified) |

However, the accuracy in the white, single blue and double blue is lower for all classes when being compared to the results of the PLS-R described in section f).

**Table 13: White/single blue/double blue classification confusion matrix of SVM-R model for single kernel**

| | | Predicted class | | |
|---|---|---|---|---|
| | | single blue | double blue | white |
| True class | single blue | 17 (70,8% classified) | 6 (25,0% misclassified) | 1 (4,2% misclassified) |
| | double blue | 8 (33,3% misclassified) | 16 (66,7% classified) | |
| | white | 2 (8,3% misclassified) | 1 (4,2% misclassified) | 21 (87,5% classified) |

The three-class model shows a 87,5% accuracy for white, a 70,8% accuracy for single blue and a 66,7% accuracy for double blue samples.

### j) Single kernel; Single blue and double blue; SVM-R

An SVM-R model trained only with single blue and double blue kernels has a lower accuracy than predicting blue and double with the three-class model as explained in section i).

**Table 14: Single blue/double blue classification confusion matrix of SVM-R model for single kernel**

| | | Predicted class | |
|---|---|---|---|
| | | single blue | double blue |
| True class | single blue | 17 (70,8% classified) | 7 (29,2% misclassified) |
| | double blue | 5 (20,8% misclassified) | 19 (79,2% classified) |

Thus, a three-class model (comprising white, single blue and double blue) shows a better performance to distinguish between single blue and double blue kernels in comparison to a model that is based just on two classes, i.e. to distinguish single blue and double blue.

In summary, the PLS-R and the SVM-R model both show that there is the potential to distinguish between white and blue samples with nearly 100% accuracy. Hence, adding extra information from NIR radiation improves existing color sorting techniques that are based on visual light only.

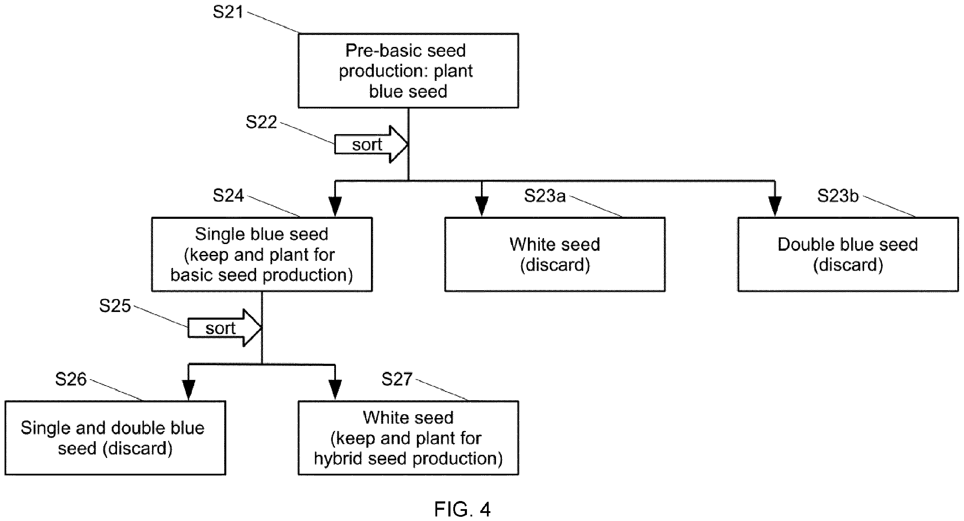

Figure 4 schematically illustrates at which stages of a seeding/harvesting cycle the present invention may be applied. In step S21, blue seed is planted during a pre-basic seed production. This results in a mix of blue and white seed being harvested. Here, the blue seed, which can be replanted for blue-white seed production, is of particular interest. In order to effectively categorize seed in the mix, the presented method for categorizing/sorting seeds is applied in step S22 and hence, a seed sorting is performed. Subsequently, white seed and double blue seed are discarded in steps S23a and S23b, respectively.

However, according to step S24, the single blue seed is kept and re-planted for blue-white basic seed production, which - again - results in a mix of blue and white seed being harvested. Here, the white seed is of interest, which will grow into male sterile seed and become the female component of hybrid seed production. Thus, a further sorting is performed in step S25 by using the inventive method for categorizing/sorting seeds.

Subsequently, blue seed, i.e. single and double blue seed, is discarded in step S26. In contrast, according to step S27, white seed is kept and planted for hybrid seed production, e.g. for the actual production of wheat.

In figure 4, the sorting in at least one of the steps S22 and S25 may be performed solely based on NIR spectroscopy but also based on a combination of NIR spectroscopy and an imaging in the visual light spectrum. Further, in at least one of the steps S22 and S25, the sorting may comprise more than one categorization cycle, wherein a categorization cycle includes NIR spectroscopy or NIR spectroscopy combined with a visual separation.

### Reference signs

- S11: Provide sample
- S12: Obtain NIR spectrum
- S13: Identify signal associated with organic colorant
- S14: Determine presence of organic colorant
- S15: Determine amount of organic colorant
- S16: Normalize amount of organic colorant
- S17: Compare normalized amount of organic colorant
- S18: Categorize bulk sample
- S19: Categorize subset of sample
- S21: Plant blue seed
- S22: Categorize/sort seeds
- S23a: Discard white seed
- S23b: Discard double blue seed
- S24: Plant single blue seed
- S25: Categorize/sort seeds
- S26: Discard single and double blue seed
- S27: Plant white seed

## Claims

1. Method for categorizing/sorting seeds, the method comprising the steps of:
providing (S11) a sample including at least one seed;
obtaining (S12) a near infrared, NIR, spectrum of at least a subset of the sample;
determining (S14) presence of an organic colorant in at least the subset of the sample based on the obtained NIR spectrum; and
categorizing/sorting (S19) at least the subset of the sample based on the determination.

2. Method according to claim 1, wherein the organic colorant comprises at least one colorant of the group of betalains, carotenoids, anthocyanins, flavonoids, anthraquinone and/or chlorophylls.

3. Method according to claim 1 or 2, further comprising the step of identifying (S13) a signal associated with the organic colorant in the obtained NIR spectrum, wherein the categorizing/sorting of at least the subset of the sample is based on the identified signal.

4. Method according to claim 3, wherein the identified signal is in the range of 15500 cm-1 to 400 cm-1.

5. Method according to claim 3, wherein the identified signal is in one of the following ranges 15385 cm⁻¹ to 10526 cm⁻¹ (650-950 nm), 8547 cm⁻¹ to 8000 cm⁻¹ (1170-1250 nm), 7519 cm⁻¹ to 7092 cm⁻¹ (1330-1410 nm), 6250 cm⁻¹ to 5917 cm⁻¹ (1600-1690 nm) and 5263 cm⁻¹ to 4167 cm⁻¹ (1900-2400 nm).

6. Method according to any one of the preceding claims, further comprising the step of:
determining (S15) an of the organic colorant in at least the subset of the sample based on the obtained NIR spectrum, wherein the categorizing/sorting (S19) of at least the subset of the sample is based on the determined amount of the organic colorant.

7. Method according to claim 6, wherein the NIR spectrum is obtained for a bulk sample, the method further comprising the steps of:
normalizing (S16) the determined amount of the organic colorant with respect to a size of the amount bulk sample;
comparing (S17) the normalized amount of organic colorant with a predefined threshold; and
categorizing/sorting (S18) the bulk sample based on the comparison.

8. Method according to any one of the preceding claims, comprising the steps of:
obtaining a first NIR spectrum of a first subset of the sample and a second NIR spectrum of a second subset of the sample;
determining presence of the organic colorant in the first subset by classifying the first NIR spectrum and in the second subset by classifying the second NIR spectrum;
categorizing/sorting the first subset and the second subset based on the classification,
wherein the classification of the NIR spectrum is performed using at least one of a principal component analysis, PCA, a partial-least-squares-regression, PLS-R, or partial least squares discriminant analysis, PLS-DA, model based on labeled spectra, a trained support vector machine classification, SVM-C, or support vector machine regression, SVM-R, model, a trained artificial neural network, ANN, or a k-nearest neighbors algorithm, k-NN.

9. Method of claim 7 or 8, further comprising the step of: sorting the first subset and the second subset based on the classification, wherein a subset of the sample consists of at least one seed.

10. Method according to any one of the preceding claims, further comprising at least one of the steps of: obtaining an image of at least the subset of the sample using at least one camera, wherein the presence of an organic colorant in at least the subset of the sample is determined based on the obtained NIR spectrum and based on the image; determining presence of an organic colorant using an X-ray based analysis; and determining presence of an organic colorant using a Raman spectroscopy analysis.

11. Apparatus for categorizing/sorting seeds comprising: a near infrared, NIR, spectrometer, including a light source configured to emit light with a wavelength in between 650 nm and 2500 nm; a detector configured to detect a NIR spectrum in a range of 15500 cm⁻¹ to 400 cm⁻¹; a sample holder configured to hold a sample relative to the light source and the detector to perform NIR measurements in a transmission and/or reflection mode; and means adapted to execute the steps of the method of any one of claims 1 to 10.

12. Apparatus according to claim 11, further comprising an image sensor configured to obtain an image of at least the subset of the sample and means adapted to execute the steps of the method of claim 10 and/or a feeder system configured to feed at least the subset of the sample to the NIR spectrometer, preferably configured to feed at least the subset of the sample to the NIR spectrometer and the image sensor.

13. Mobile device comprising the apparatus according to claim 11 or 12.

14. Use of the method according to any one of claims 1 to 10 and/or the apparatus according to any one of claims 11 or 12 for categorizing/sorting seeds of a hybrid seed system, the hybrid seed system comprising seeds including an organic colorant.

15. Use according to claim 14, wherein the hybrid seed system is a hybrid wheat system comprising a monosomic alien addition chromosome carrying a male fertility restorer gene and a color marker gene as a selection marker gene and wherein the color marker gene is able to confer a characteristic coloration and is co-expressed with a fertility-related gene.
